# EUROPEAN PATENT APPLICATION

(11) **EP 3 664 122 A1**
(43) Date of publication of application: **10.06.2020**
(21) Application number: 19213489.8
(22) Date of filing: 04.12.2019
(51) Int. Cl.: H01J 37/32, A61B 18/04, A61L 2/14

(54) **A METHOD TO CONTROL A COLD PLASMA TREATMENT PROCESS FOR CLEANING, DISINFECTION AND STERILIZATION PURPOSES OF AN OBJECT**

(30) Priority: 05.12.2018 NL 2022138
(71) Applicant: Blue Plasma Holding B.V., 5928 RC Venlo (NL)
(72) Inventor: Lauret, Menno, 5928 RC Venlo (NL); Sobota, Anna, 5612 AP Eindhoven (NL); Van Keulen, Thijs Adriaan Cornelis, 5616 TT Eindhoven (NL); Van Rijsingen, Eva Anne Barbara, 5928 RC Venlo (NL)
(74) Representative: Veldman-Dijkers, Cornelia G. C.

(57) **Abstract**

By a method to control a cold plasma treatment process for cleaning, disinfection and sterilization purposes of an object, a cold plasma is being generated based on at least one input variable. The cold plasma comprises at least one plasma variable. The cold plasma is being used to treat an object to change at least one object variable of said object. At least a relationship between an input and an output is assumed to be a priori unknown, nonlinear, time-varying and having an optimum. It is known whether said optimum is a minimum or maximum. A control algorithm is being used to find said optimum.

## Description

### FIELD OF THE INVENTION

The invention relates to a method to control a cold plasma treatment process for cleaning, disinfection and sterilization purposes of an object, whereby based on at least one input variable a cold plasma is being generated, which cold plasma comprises at least one plasma variable, wherein said plasma is being used to treat an object to change at least one object variable of said object.

### BACKGROUND OF THE INVENTION

Objects like solid objects, liquids or gasses can be treated with plasma, like cold plasma, for example for cleaning, disinfection and sterilization purposes. Such purposes include antimicrobial, antiviral, antifungal treatment of an object. Cold plasma is also referred to as "non-thermal plasma" being non-equilibrium ionized gases, with two temperatures: ions and neutrals stay at a low temperature (sometimes room temperature), whereas electrons are much hotter.

The cold plasma treatment process can be used for example in the agro-food business, for medical purposes etc. Plasma cleaning is the removal of impurities and contaminants from for example surfaces through the use of plasma created from gaseous species. Gases such as argon and oxygen, as well as mixtures such as air and hydrogen/nitrogen can be used. Plasmas are created by using high frequency voltages (typically kHz to >MHz) to ionise a low-pressure gas (typically around 1/1000 atmospheric pressure), although atmospheric pressure plasmas are also common. To optimize the plasma treatment process, one would like to optimize the plasma. However, in plasma different physical and chemical plasma processes occur on a variety of very short time-scales (nanoseconds to second) compared to the time scale of the total treatment (beings seconds to minutes). Furthermore said processes tend to change if circumstances influencing the plasma (e.g. air temperature or humidity) change. No easy-to-use model taking the physical and chemical plasma processes as well as the changes of these processes due to a change in circumstances into account exist. Cold plasma sources typically create rather unpredictable effects (e.g. the concentrations of reactive species are different for identical experiments or change due to changing temperature or humidity) due to the complex and strongly nonlinear nature of the cold atmospheric plasma and its sensitivity to changing circumstances.

This renders it very difficult to control the plasma and therefore the outcome of the plasma treatment process. The difficulty to control the outcome of an atmospheric plasma treatment is a major obstacle to widespread implementation of plasma technology for the applications mentioned. This is due to both the unpredictability of the process result itself and the resulting reduced economic benefits that can be achieved.

### SUMMARY OF THE INVENTION

At least one of the objects of the invention is to provide a method to control a plasma treatment process of an object, despite the lack of a predictive model of the plasma suitable for control methods.

This object is accomplished with the method according to the invention in that at least a relationship
- between an input comprising the at least one input variable and an output comprising the at least one plasma variable or a function thereof or the at least one object variable or a function thereof or
- between an input comprising the at least one plasma variable and an output comprising the at least one object variable or a function thereof
is assumed to be a priori unknown, nonlinear, time-varying and having an optimum, whereby it is known whether said optimum is a minimum or maximum, whilst a control algorithm is being used to find said optimum, which control algorithm comprises the following steps:
A) estimating in real time at a current value of the input a gradient between on the one hand the current value of the input and a perturbation of the current value of the input and on the other hand a current value of the output as result of the current value of the input and the value of the output as result of the perturbation of the current value of the input,
B) computing a new value for the input by a gradient descent if the optimum is a minimum and a gradient ascent if the optimum is a maximum,
C) repeating the steps A) and B) until the optimum has been found.

Input variables are for example voltage and frequency of a high-voltage source, the flow of the gas to create plasma etc.

Plasma variables are for example the concentration of active plasma species (like ozone or radicals like OH⁻).

It has been established by the applicant that on a time-scale relevant for the typical plasma treatment applications (longer than one second), the effect of these input on the relevant plasma variables (e.g. concentration of chemical species) can be modelled by a measured nonlinear relationship. This relationship has one optimum (either maximum or minimum) and typically changes only slowly in time (compared to the time-scale of the plasma treatment process).

Object variables are for example the amount of bacteria on or in the object. Also the relationship between the input and the object variables can be modelled by a measured nonlinear relationship one optimum and typically changes only slowly in time.

So, although no exact model of the relationship is available and is thus a priori unknown, it is assumed to be nonlinear, time-varying and having an optimum. It is assumed whether this optimum is a maximum or a minimum.

According to the method a value of the output belonging to a current value of the input is being measured or estimated. Then a value of another output belonging to a perturbation of the current value of the input is being measured or estimated. Based on these two sets of values, a gradient is calculated.

In case that the optimum is a maximum and the gradient is positive, a new value for the input will be the current input plus the perturbation. If the gradient is negative, a new value for the input will be the current input minus the perturbation. This is called gradient ascent.

In case that the optimum is a minimum and the gradient is positive, a new value for the input will be the current input minus the perturbation. If the gradient is negative, a new value for the input will be the current input plus the perturbation. This is called gradient descent.

These steps will be repeated until the maximum or minimum has been found, or until a desired output level has been reached.

The nonlinear relationship between input and output tends to change if circumstances influencing the plasma (e.g. air temperature or humidity) change. This causes the desired optimum to be a priori unknown and also to slowly change over time. The change of the optimum is on a larger time scale (seconds to minutes) than the change in the different physical and chemical plasma processes which occur on a variety of very short time-scales (nanoseconds to second). The method of the invention focuses on the time-scale that is relevant for the specific application, ignoring (averaging out) very short time-scale changes that do not affect the application result.

If possible, it is best to measure the effect of the treatment so to control the value of the object variable. If this is not possible then a relationship between plasma and the treatment has to be known or assumed and the value of the at least one plasma variable like concentration of plasma species has to be controlled to a value that is assumed or has been found to lead to a good treatment. Note that it is possible that one would need to maximize a plasma variable like concentration (for example ozone or radicals like OH⁻) to minimize the result of the treatment and the value of the object variable (for example amount of bacteria). Note that all kind of relations between plasma and treatment effect can occur. If it is also not possible to measure the active plasma species (like ozone or radicals like OH⁻) in real-time, then it is possible to measure the electric variables of the high voltage circuit: voltage, current and indirectly the dissipated power. These variables (especially the dissipated power) typically have a correlation with the active plasma species. Therefore, by controlling and optimizing one or more of these electric variables it is possible to indirectly control and optimize the active species in the plasma and hence the functional effect of the plasma on the object.

The control method can be used to optimize plasmas for efficiency and/or promote specific plasma chemistry processes.

The control method can also makes the plasma less sensitive and more robust for disturbances and changing circumstances.

It has to be noticed that WO0225695A2 is directed to etching and deposition of materials. It is directed to improve plasma process uniformity, whereas the present invention is directed to optimize plasma treatment process, whereby the plasma process does not need to be uniform.

XP000558395 Rietman E.A. is directed to hot plasma etching and not to control a cold plasma treatment process for cleaning, disinfection or antimicrobial purposes.

An embodiment of the method according to the invention is characterized in that the control algorithm is an extremum seeking algorithm.

By means of such extremum seeking algorithm the optimum can easily be found. Contrary to most control algorithms, such an extremum seeking algorithm can find the optimum for an unknown process and for a slowly changing optimum.

Another embodiment of the method according to the invention is characterized in that in case a constant predetermined value of the at least one plasma variable or the at least one object variable is desired as result of the input, the output is said function of the at least one plasma variable or the at least one object variable respectively, whereby the function comprises a cost function.

The method of the invention is directed to find an optimum, being either a minimum or maximum, between a relation of the input and output. A constant predetermined value of the at least one plasma variable or the at least one object variable as result of the input, is not an optimum. To still be able to use the method according to the invention, a function needs to be used based on the at least one plasma variable or the at least one object variable respectively, which function need to have an optimum. Such a function is for example a cost function

Another embodiment of the method according to the invention is characterized in that the perturbation of the current value of the input comprises a dither-like, periodic disturbance like a∗sin(f∗t)), with "a" being amplitude, "f" being frequency and "t" being time, wherein the duration of one disturbance is shorter than the time needed for the output to react on a change of the input.

The time-scale of the dither-like, periodic disturbance is shorter than the adaptation time-scale in which the input changes.

Based on such input the output will have a form b∗sin(f∗t+c)), with "b" being amplitude, "f" being frequency , "t" being time and "c" being a delay time.

Based on these inputs and outputs and if needed some filtering, one can obtain an estimation of the gradient.

It is of course also possible to use another periodic disturbance like a cosine,

Another embodiment of the method according to the invention is characterized in that the perturbation of the current value of the input comprises a relatively small change of the value of the input compared to a total input range over a relatively small time step compared to a total treatment time resulting in a change of the value of the output whereby the gradient is being estimated by taking the ratio of the change of the value of the output to change of the value of the input over several time steps.

Estimating the gradient over several time steps leads to a filtered gradient estimate. This approach has the benefit that no disturbing dither is needed, that noise is automatically filtered, and that tuning becomes easier.

Another embodiment of the method according to the invention is characterized in that the output is being measured or estimated.

Preferably the output is being measured. If this is not possible the output can be estimated, for example based on earlier measurements and experiments.

Another embodiment of the method according to the invention is characterized in that the cold plasma is a cold atmospheric plasma.

Such a cold atmospheric plasma is created in ambient air, which has the advantages that no specific arrangements to create a desired pressure temperature or other forms of preconditioning of the gas need to be made.

### BRIEF DESCRIPTION OF THE DRAWINGS

The method according to the invention will further be explained with reference to the drawings, wherein,
figures 1A and 1B are graphs showing the amount of ozone in a plasma in time by a prior art plasma treatment process,
figure 2 is a schematic view of a first embodiment of a control system using the method according to the invention,
figures 3A and 3B are graphs showing the amount of ozone in a plasma in time by a prior art plasma treatment process,
figure 4 is a schematic view of a second embodiment of a control system using the method according to the invention.

In the drawings, like reference numerals refer to like elements.

### DESCRIPTION OF PREFERRED EMBODIMENTS

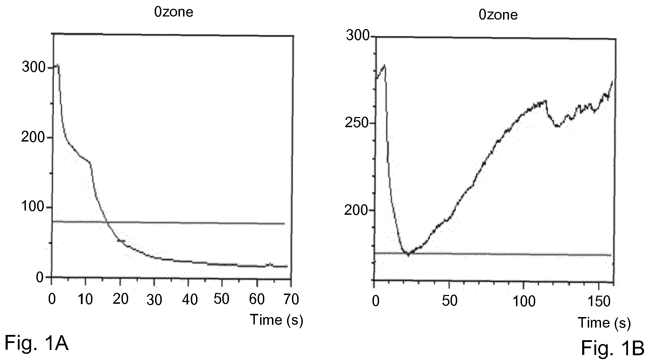

Figures 1A and 1B are graphs showing the amount of ozone in a cold plasma in time by a prior art plasma treatment process. On the x-axis the time is given in seconds, on the y-axis the amount of ozone is given in arbitrary units.

Both measurements as shown in figure 1A and 1B respectively were made with the same high-voltage source having identical settings, with the same room temperature and pressure. However, apparently due to some unknown circumstances the amount of ozone by both measurements are completely different.

It has been concluded, also based on further measurements that cold plasma sources typically create rather unpredictable effects (e.g. the concentrations of reactive species are different for identical experiments) due to the complex and strongly nonlinear nature of the cold atmospheric plasma and its sensitivity to changing circumstances like a change in temperature, humidity or other unknown features.

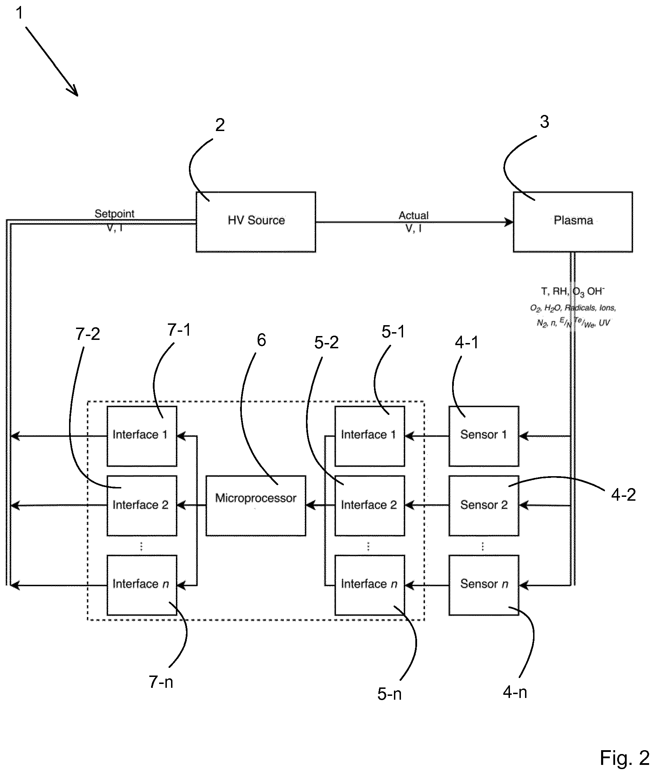

Figure 2 is a schematic view of a first embodiment of a control system 1 using the method according to the invention.

The control system 1 comprises a high voltage source 2 for generating a plasma 3, a plasma 3, a number of sensors 4-1, 4-2, .., 4-n, a number of interfaces 5-1, 5-2, ..., 5-n to convert the values as measured by the sensors 4-1, 4-2, .., 4-n into values to be entered into a microprocessor 6, a number of interfaces 7-1, 7-2, ..., 7-n to convert values as calculated by the microprocessor 6 into values to be entered into the high voltage source 2.

The high voltage source 2 is provided with a voltage V and current I as actual values to generate plasma 3. These actual values are considered to be the input values and form the input.

The plasma 3 comprises a number of plasma species like ozone, radicals, ions and other species. The amount of all or some of these plasma species can be measured by means of the sensors 4-1, 4-2, .., 4-n. By means of the interfaces 5-1, 5-2, ..., 5-n these values are converted into values to be entered into a microprocessor 6. The values to be entered into a microprocessor 6 are considered to be the output values and form the output. Such control system 1 can be used in case that a relation between plasma 3 and the result of treating an object 8 with such plasma is known.

Based on for example earlier experiments it is concluded that the exact relationship between the input and the output is as such unknown, but that the relationship nonlinear, time-varying and has an optimum. It is also concluded that said optimum is a maximum or minimum.

By means of the microcomputer 6 a control algorithm is being used to find said optimum. With the control algorithm the following steps are being taken.

In a first step in real time at a current value of the input a gradient between on the one hand the current value of the input and a perturbation of the current value of the input and on the other hand a current value of the output as result of the current value of the input and the value of the output as result of the perturbation of the current value of the input is being estimated or calculated. Then in a second step a new value for the input is being calculated based on a gradient descent if the optimum is a minimum and based on a gradient ascent if the optimum is a maximum is being calculated. The new input is converted by means of the interface 7 into set points for the values V and I for the high voltage source 2. The first and second steps are being repeated until the optimum has been found.

The control algorithm can be an extremum seeking algorithm.

The perturbation of the current value of the input may comprise a dither-like, periodic disturbance like a∗sin(f∗t)), with "a" being amplitude, "f" being frequency and "t" being time, wherein the duration of one disturbance is shorter than the time needed for the output to react on a change of the input.

The perturbation of the current values of V and I of the input may also comprises a relatively small change of the value of the input compared to a total input range for V and I over a relatively small time step, for example seconds compared to a total treatment time, for example several minutes, resulting in a change of the value of the output whereby the gradient is being estimated by taking the ratio of the change of the value of the output to change of the value of the input over several time steps.

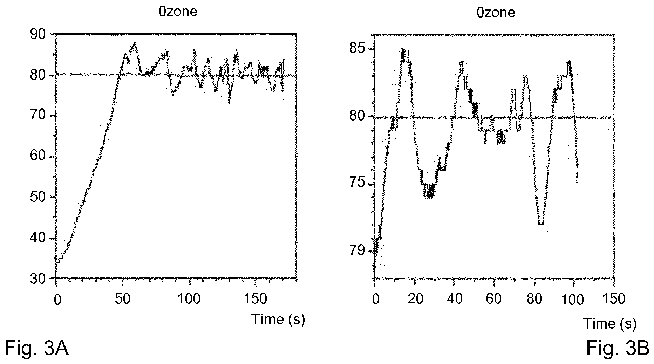

Figures 3A and 3B are graphs showing the amount of ozone in plasma 3 in time when using the control system 1, whereby an amount of 80 in the plasma 3 is desired. On the x-axis the time is given in seconds, on the y-axis the amount of ozone is given in arbitrary units. In such a case whereby a constant predetermined value of the plasma variable of 80 is desired, the output as entered into the microprocessor 6 is a function of the plasma variable, whereby the function comprises a cost function. The cost function is a convex or concave function preferably with a unique optimum, for example V= (y-80) ^ 2.

These measurements were made with the same high-voltage source 2 having identical starting settings, with the same room temperature and pressure. As can be seen the amount of ozone by these two measurements differ substantially in the beginning of the treatment process but due to the method according to the invention, after 60 seconds the amount of ozone is by both measurements in a relatively small bandwidth from 75-85.

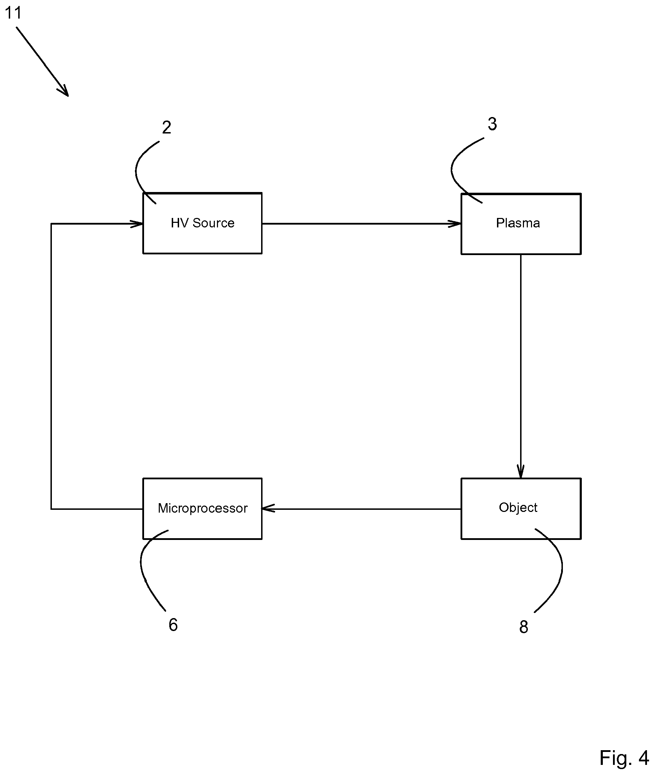

Figure 4 is a schematic view of a second embodiment of a control system 11 using the method according to the invention.

The control system 11 comprises a high voltage source 2 for generating a plasma 3, a plasma 3, an object 8 to be treated with the plasma 3, and a microprocessor 6. By means of sensors in a manner similar as by first embodiment, object values like the amount of bacteria can be measured by means of sensors. By means of interfaces these values are converted into values to be entered into a microprocessor 6. The values to be entered into a microprocessor 6 are considered to be the output values and form the output. The same method as by first embodiment is being used in the microprocessor 6.

Since the purpose of the plasma treatment is to treat the object 8, it is preferred to measure the object values, if this is possible.

It is also possible that the perturbation of the current value of the input comprises a dither-like, non-periodic disturbance.

### LIST OF REFERENCE SIGNS

- 1: control system
- 2: high voltage source
- 3: plasma
- 4: sensor
- 5: interface
- 6: microprocessor
- 7: interface
- 8: object
- 11: control system

## Claims

1. A method to control a cold plasma treatment process for cleaning, disinfection and sterilization purposes of an object, whereby based on at least one input variable a plasma is being generated, which cold plasma comprises at least one plasma variable, wherein said cold plasma is being used to treat an object to change at least one object variable of said object, **characterized in that** at least a relationship
- between an input comprising the at least one input variable and an output comprising the at least one plasma variable or a function thereof or the at least one object variable or a function thereof or
- between an input comprising the at least one plasma variable and an output comprising the at least one object variable or a function thereof
is assumed to be a priori unknown, nonlinear, time-varying and having an optimum, whereby it is known whether said optimum is a minimum or maximum, whilst a control algorithm is being used to find said optimum, which control algorithm comprises the following steps:
A) estimating in real time at a current value of the input a gradient between on the one hand the current value of the input and a perturbation of the current value of the input and on the other hand a current value of the output as result of the current value of the input and the value of the output as result of the perturbation of the current value of the input,
B) computing a new value for the input by a gradient descent if the optimum is a minimum and a gradient ascent if the optimum is a maximum,
C) repeating the steps A) and B) until the optimum has been found.

2. A method according to claim 1, **characterized in that** the control algorithm is an extremum seeking algorithm.

3. A method according to claim 1 or 2, **characterized in that** in case a constant predetermined value of the at least one plasma variable or the at least one object variable is desired as result of the input, the output is said function of the at least one plasma variable or the at least one object variable respectively, whereby the function comprises a cost function.

4. A method according to claim 1, 2 or 3, **characterized in that** the perturbation of the current value of the input comprises a dither-like, periodic disturbance like a∗sin(f∗t)), with "a" being amplitude, "f" being frequency and "t" being time, wherein the duration of one disturbance is shorter than the time needed for the output to react on a change of the input.

5. A method according to claim 1, 2 or 3, **characterized in that** the perturbation of the current value of the input comprises a relatively small change of the value of the input compared to a total input range over a relatively small time step compared to a total treatment time resulting in a change of the value of the output whereby the gradient is being estimated by taking the ratio of the change of the value of the output to change of the value of the input over several time steps.

6. A method according to one of the preceding claims, **characterized in that** the output is being measured or estimated.

7. A method according to one of the preceding claims, **characterized in that** the cold plasma is a cold atmospheric plasma.
